# EUROPÄISCHE PATENTSCHRIFT

(11) **EP 1 296 939 B1**
(45) Veröffentlichungstag und Bekanntmachung des Hinweises auf die Patenterteilung: **09.11.2005**
(21) Anmeldenummer: 01947442.8
(22) Anmeldetag: 04.07.2001
(51) Int. Cl.: C07C 249/08, C07C 251/60, C07C 49/12

(54) **VERFAHREN ZUR HERSTELLUNG VON TRIOXIMDERIVATEN**
METHOD FOR THE PRODUCTION OF TRIOXIME DERIVATIVES
PROCEDE DE PRODUCTION DE DERIVES DE TRIOXIMES

(30) Priorität: 05.07.2000 DE 10032031
(43) Veröffentlichungstag der Anmeldung: 02.04.2003
(73) Patentinhaber: BASF Aktiengesellschaft, 67056 Ludwigshafen (DE)
(72) Erfinder: KRAMER, Andreas, 67098 Bad Dürkheim (DE); KIEFER, Matthias, 69226 Nussloch (DE); HENNINGSEN, Michael, 67227 Frankenthal (DE); SIEGEL, Wolfgang, 67117 Limburgerhof (DE); SCHRÖDER, Jochen, 67245 Lambsheim (DE)
(86) Internationale Anmeldenummer: PCT/EP2001/007660
(87) Internationale Veröffentlichungsnummer: WO 2002/002514

(56) Entgegenhaltungen:
- WO-A-00/18726
- H. PIELARTZIK ET AL.: "Methoden der organischen Chemie" 1985 , GEORG THIEME , STUTTGART-NEW YORK XP002183768 in der Anmeldung erwähnt Seite 780 -Seite 781
- H. METZGER ET AL.: "Methoden der organischen Chemie" , GEORG THIEME , STUTTGART- NEW YORK XP002183769 in der Anmeldung erwähnt Seite 217 -Seite 219
- G. PONZIO ET AL.: "Ricerche sulle diossime (II)" GAZZ.CHIM.ITAL., Bd. 22, 1922, Seiten 289-301, XP001022657 in der Anmeldung erwähnt

## Beschreibung

Die vorliegende Erfindung betrifft ein Verfahren zur Herstellung zur Herstellung von Trioximderivaten der Formel I, 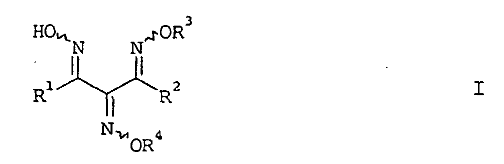 in der die Substituenten R¹ und R² gleich oder verschieden sein können und jeweils Cyano, C₁-C₄-Alkyl, C₁-C₄-Halogenalkyl und C₃-C₆-Cycloalkyl und R³ und R⁴ gleich oder verschieden sein können und jeweils C₁-C₄-Alkyl, C₁-C₄-Halogenalkyl, C₃-C₆-Cycloalkyl, C₁-C₄-Alkylcarbonyl und Aryl-C₁-C₄-alkyl bedeuten können,
durch folgende Reaktionsschritte in Wasser ohne Isolierung der Zwischenprodukte:
A) Nitrosierung von 1,3-Diketonen der Formel II 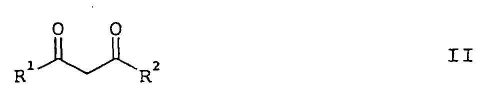 zu Monooximen der Formel III, 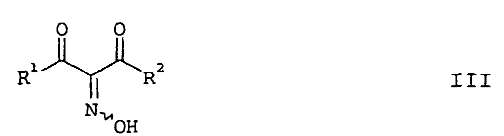
B) Oximierung von III mit Hydroxyaminderivaten der Formel IV

   H₂N-OR^{3'} IV

   in der R^{3'} für Wasserstoff oder eine Gruppe R³ steht, oder deren Säureadditionssalzen, zu Bisoximen der Formel V, 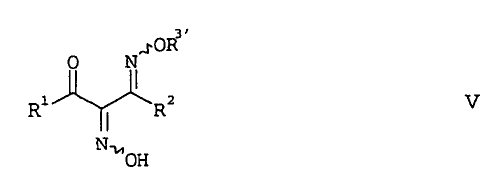
C) Alkylierung von V mit Alkylierungs- oder Acylierung von V mit Acylierungsmitteln zu Verbindungen der Formel VI und 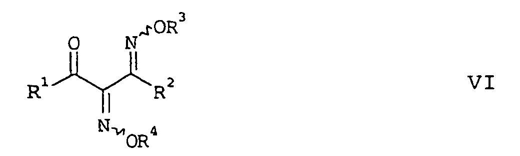
D) anschließender Oximierung von VI mit Hydroxylamin zu Verbindungen der Formel I.

Verfahren zur Herstellung von Trioximderivaten der Formel (I) ist aus WO 00/18726 durch Bisoximetherketal als Zwischenprodukt bekannt.

Zur Synthese von Oximen und O-Alkyl-Oximethern sind im Stand der Technik verschiedene Methoden bekannt [Houben-Weyl, Methoden der organischen Chemie, 4. Aufl., Bd. 10/4, S. 17ff., 55ff., 73ff., 217ff., Thieme Verlag Stuttgart und New York (1979)].

Alkylierungsverfahren für aktivierte Oxime sind ebenfalls im Stand der Technik bekannt.

In Gazz. Chim. Ital., Bd. 22, S. 289ff. (1922) wird die Synthese von Pentan-2,3,4-trioxim und dessen Benzylierung zur Trisbenzyloximether beschrieben.

Die Synthese von Pentan-2,3,4-trioxim aus Isonitrosoacetylaceton mit Hydroxylammoniumchlorid in Wasser ist aus Rev. Acad. Cienc. Exactas, Fis.-Quim. Nat. Zaragoza, Bd. 31, S. 91-100 (1976) bekannt.

In Bull. Acad. Sci. USSR, Div. Chem. Sci. (Engl. Transl.), EN, Bd. 28, S. 121-128 (1979) werden Alkylierungsreaktionen von Oximen durch Diazoalkane, Alkylhalogenide und Dialkylsulfate beschrieben; insbesondere die Konkurrenz zwischen Sauerstoff- und Stickstoffalkylierung wird untersucht. Die Herstellung der O-A1-kylether von α,α'-Bis-carbonyloximen in Aceton, Ethanol, Wasser oder Diethylether wird in mäßigen Ausbeuten beschrieben. Zur Begünstigung der O-Alkylierung wird empfohlen, bei möglichst hohen Temperaturen zu arbeiten, da die Nitrone temperaturlabil sind, oder möglichst voluminöse Alkylierungsreagenzien zu verwenden.

In Ind. J. Chem., Bd. 30B, S. 749-753 (1991) wird die Methylierung der Oxime der Formel III, in der R¹ und R² für Methyl oder Phenyl stehen, beschrieben. Aus der Umsetzung mit Methyliodid in Gegenwart von wasserfreiem K₂CO₃ in Aceton werden die O-Methylether in Ausbeuten von 65, bzw. 67 % erhalten.

Aufgabe der vorliegenden Erfindung war es, ein wirtschaftliches, sicherheitstechnisch unbedenkliches und großtechnisch anwendbares Verfahren zur Herstellung von O-Alkyl- oder O-Acyloximethern der Formel I bereitzustellen, das die Endprodukte mit hoher Selektivität und Ausbeute zugänglich macht und in dem keine Lösungsmittelwechsel oder Isolierung von Zwischenstufen notwendig sind.

Demgemäß wurde das eingangs definierte Verfahren gefunden.

Durch das erfindungsgemäße Verfahren wird die Aufgabe gelöst, da sämtliche Syntheseschritte in Wasser als Lösungsmittel durchgeführt werden, ohne daß Zwischenprodukte abgetrennt, isoliert oder aufgereinigt werden müssen. Dem Wasser können auch geringe Mengen (bis zu 50 Vol.-%, insbesondere bis zu 10 Vol.-%) inerter, mit Wasser mischbarer organischer Lösungsmittel zugesetzt werden, um im Fall schwer löslicher Ausgangsverbindungen, Zwischen- oder Endprodukte den Reaktionsverlauf zu begünstigen.

In einer bevorzugten Ausführungsform des erfindungsgemäßen Verfahrens erfolgt kein Zusatz organischer Lösungsmittel.

Die Reaktionen der Schritte A bis D sind an sich in der Literatur bekannt, sie werden üblicherweise in organischen Lösungsmitteln, wie aromatische Kohlenwasserstoffen, Ether oder Alkoholen durchgeführt. Es ist jedoch nicht bekannt, daß Bis- und Trisoxime und entsprechende -oximether in Wasser als Lösungsmittel auf diesem Weg mit so hoher Selektivität herstellbar sind, daß eine Isolierung der Zwischenprodukte unnötig ist.

In der ersten Stufe (Schritt A) des Verfahrens werden 1,3-Diketone der Formel II nitrosiert. Die Nitrosierung wird unter sauren Bedingungen mit C₁-C₄-Alkylnitriten [vgl. Chem. Ber., Bd. 20, S. 252 und S. 656 (1887)] oder Alkali- oder Erdalkalinitriten [vgl. J. Chem. Soc., S. 3052 (1957)] oder aber mit Stickstoffoxid (NO) und Luftsauerstoff durchgeführt [vgl. Chem. Ber., Bd. 37, S. 1524 (1904)]. Die Verwendung von Alkali- oder Erdalkalinitriten, insbesondere Kalium- oder Natriumnitrit, bei pH-Werten ≤ 5 ist bevorzugt. Die Art der Säure hat üblicherweise keinen Einfluß, aus praktischen Gründen stellt man den pH-Wert durch Zugabe von anorganischen Säuren, wie Salz- oder Schwefelsäure, ein.

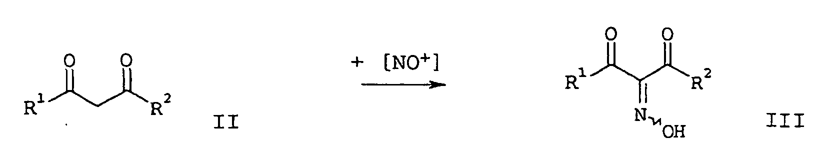

Diese Umsetzung erfolgt üblicherweise bei Temperaturen von -10°C bis +120°C, vorzugsweise 0°C bis 80°C in Gegenwart einer Säure.

Die Edukte werden im allgemeinen in äquimolaren Mengen miteinander umgesetzt. Es kann für die Ausbeute vorteilhaft sein, das Nitrit in einem Überschuß bezogen auf II einzusetzen.

1,3-Diketone der Formel II sind kommerziell erhältlich.

In der zweiten Stufe (Schritt B) werden die Monooxime der Formel III mit Hydroxyaminderivaten der Formel IV zu Bisoximen der Formel V umgesetzt.

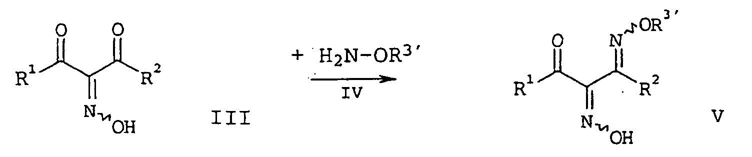

In Formel IV bedeutet R^{3'} Wasserstoff oder eine Gruppe R³. Die Umsetzung kann in einer Ausführungsform des Verfahrens mit Hydroxyaminethern, in denen R^{3'} gleich R³ ist, direkt zu den Bisoximmonoethern V geführt werden.

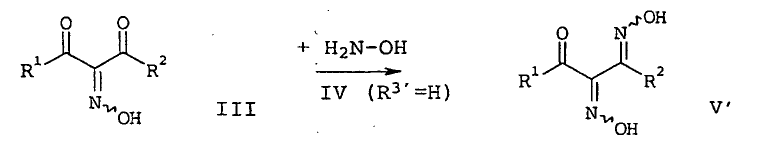

In einer anderen Ausführungsform des Verfahrens wird mit Hydroxyamin der Formel IV (R^{3'} = H) zu Bisoximen V' umgesetzt und erst in dem nächsten Schritt C) beide Oximgruppen alkyliert oder acyliert. In dieser Ausführungsform sind nur Verbindungen zugänglich, bei denen R³ und R⁴ gleich sind.

In beiden Ausführungsformen ist die Reaktion unabhängig von dem pH-Wert. Aus praktischen Gründen wird die Reaktion üblicherweise unter den selben oder ähnlichen pH-Bedingungen durchgeführt wie die erste Stufe.

In beiden Ausführungsformen können die Hydroxylaminderivate der Formel IV entweder als freie Basen oder als Säureadditionssalze eingesetzt werden. Aus praktischen Gründen wird häufig die Verwendung der entsprechenden Hydrochloride oder -sulfate in Betracht gezogen.

Die Umsetzung von III mit IV erfolgt üblicherweise bei Temperaturen von -10°C bis 120°C, vorzugsweise 0°C bis +120°C.

In der dritten Stufe (Schritt C) werden die Bisoximmonoether V, bzw. die Bisoxime V' mit Alkylierungs- oder Acylierungsmittel in die Bisoximdiether VI überführt.

Als Alkylierungsmittel kommen C₁-C₄-Alkylhalogenide, C₁-C₄-Halogenalkylhalogenide, C₃-C₆-Cycloalkylhalogenide, C₁-C₄-Alkylcarbonylhalogenide und Aryl-C₁-C₄-alkylhalogenide oder auch die entsprechenden Sulfonate oder p-Toluolsulfonate oder Dialkylcarbonate, Dialkylsulfate, insbesondere Dimethylsulfat in Betracht. Aus der Gruppe der Halogenide sind die Bromide und Chloride bevorzugt.

Als Acylierungsmittel kommen Halogenide, insbesondere Chloride von C₁-C₄-Alkylcarbonsäuren oder entsprechende Anhydride in Betracht. Bevorzugt ist die Verwendung von Acetylchlorid und Acetanhydrid.

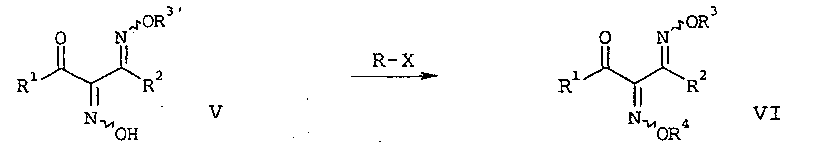

In dem vorstehenden Reaktionsschema steht "R" für eine Gruppe R³ und/oder R⁴ mit der Bedeutung C₁-C₄-Alkylcarbonyl und "X" für eine Abgangsgruppe wie Halogen oder Alkylcarbonyloxy.

Diese Umsetzung erfolgt üblicherweise bei Temperaturen von -10°C bis +120°C, vorzugsweise 0°C bis 70°C in Gegenwart einer Base, so daß eine zumindest teilweise Deprotonierung der Oxim-Gruppen erfolgt. Die Reaktion wird vorteilhaft bei pH-Werten von 7-14, insbesondere von pH 10-14 durchgeführt.

Das erfindungsgemäße Verfahren ist nicht auf bestimmt substituierte Verbindungen beschränkt, sofern die Substituenten unter den Reaktionsbedingungen inert sind. Aliphatische Reste können geradkettig oder verzweigt sein. Die Kettenlänge der Substituenten ist für das erfindungsgemäße Verfahren ohne Belang, jedoch wird man aus technischen Gründen üblicherweise Reste mit höchstens 4 C-Atomen wählen.

Ganz besonders bevorzugt wird das Verfahren zur Herstellung von Pentan-2,3,4-trion-3,4-O-methyloxim verwendet.

Die Substituenten R¹ und R² können weitere, unter den Reaktionsbedingungen inerte Reste tragen, dabei seien beispielhaft genannt: Halogen, Cyano, SO₃H, COOH, COOR^{b}, Alkyl, Alkenyl, Alkinyl, Aryl oder Heteroaryl; R^{b} bedeutet C₁-C₁₀-Alkyl.

Alkyl steht generell für C₁-C₁₀-Alkyl, insbesondere für C₁-C₄-Alkyl, das gilt auch für Halogenalkyl; Cycloalkylreste haben drei bis sechs Ringglieder. Aryl steht beispielsweise für Phenyl oder Naphthyl.

Heteroaryl bedeutet beispielsweise Furyl, Thienyl, Pyrrolyl, Isoxazolyl, Isothiazolyl, Pyrazolyl, Oxazolyl, Imidazolyl, Pyridyl, Pyridazinyl, Pyrimidinyl oder Triazinyl;

Halogen bedeutet Chlor, Fluor, Brom oder Jod.

Alkenyl- und Alkinylreste haben zwei bis acht, insbesondere drei bis sechs C-Atome.

Die nach dem erfindungsgemäßen Verfahren erhältlichen O-Alkyloximether eignen sich als Zwischenprodukte zur Herstellung von Farbstoffen oder Wirkstoffen auf dem Pharma- oder Pflanzenschutzbereich.

Die nachfolgenden Beispiele sollen das erfindungsgemäße Verfahren weiter erläutern:

### Beispiele:

### Schritt A): Synthese von Acetylhydroxyiminoaceton

Eine Lösung von 69,0 g (1 Mol) NaNO₂ in 103 g Wasser wurden mit 50 Gew.-%iger H₂SO₄ auf pH 4,0 bis 4,6 eingestellt. Bei etwa 15 °C wurde ein Gemisch aus 100,0 g (1 Mol) Acetylaceton und 475 g Wasser während 105 min bei konstantem pH zudosiert. Während der Reaktion entstand eine produkthaltige organische Phase.

Nach gaschromatographischer Analyse war der Umsatz von Acetylaceton quantitativ.

Die Reaktionslösung wurde ohne Reinigung in die nächste Stufe eingesetzt.

### Schritt B): Synthese von Pentan-2,3,4-trion-(3-O-methyloxim)-4-oxim

Die Reaktionslösung der 1. Stufe wurde bei etwa 20 bis 25 °C während etwa 2 Std. mit 306,0 g einer 30 Gew.-%igen wässrigen Lösung von Methoxyaminhydrochlorid versetzt. Der pH wurde durch Zulauf, von 25 Gew.-% NaOH-Lösung bei 4,0 bis 4,6 gehalten. Nach 3 Std. wurde die ölige produkthaltige Phase gaschromatographisch analysiert. Die verschiedenen Isomeren der Titelverbindung machten im Gaschromatogramm etwa 83 Flächen-% aus.

Die Reaktionslösung wurde ohne Reinigung in die nächste Stufe eingesetzt.

### Schritt C): Methylierung zu Pentan-2,3,4-trion-3,4-bis(O-methyloxim)

Die Reaktionslösung der 2. Stufe wurde durch 25 Gew.-% NaOH-Lösung auf pH 10 bis 11 eingestellt; bei diesem pH wurden 138,7 g (1,1 Mol) Dimethylsulfat bei etwa 20 bis 25 °C während etwa 90 min zugegeben, diee Lösung weitere 90 min gerührt.

Die Reaktionslösung wurde ohne Reinigung in die nächste Stufe eingesetzt.

### Schritt D): Synthese von Pentan-2,3,4-trion-3,4-bis(O-methyloxim)-2-oxim

Die Reaktionslösung der 3. Stufe wurde nach Einstellen durch 25 Gew.-% NaOH-Lösung auf pH 13 bis 14 bei etwa 20 bis 25 °C während etwa 60 bis 75 min mit 106,7 g (1,1 Mol) einer 30 Gew.-%igen wässrigen Lösung von Hydroxylammoniumsulfat bei konstantem pH versetzt. Nach einer weiteren Stunde Rühren wurde schwach angesäuert (auf pH 6) und die Reaktionslösung mit Methyl-tert.Butylether (MTBE) extrahiert. Nach Waschen der organischen Phase mit Wasser und Trocknen wurde das Lösungsmittel entfernt.

Es wurden 177 g der Titelverbindung erhalten.

Nach HPLC-Analyse betrug die Ausbeute bezogen auf eingesetztes Acetylaceton 62 bis 63 % der Theorie. Das Rohprodukt enthielt nach HPLC-Analyse mit internem Standard folgende Isomere (Gew.-%)

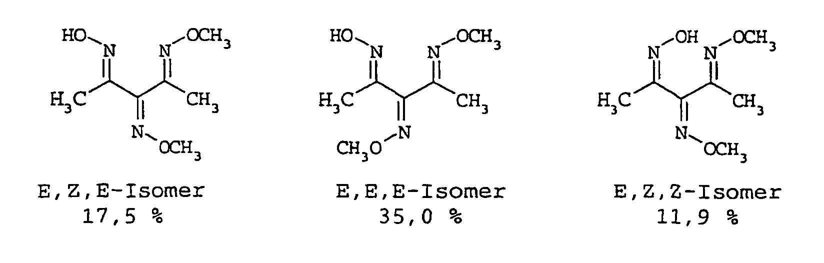

## Patentansprüche

1. Verfahren zur Herstellung von Trioximderivaten der Formel I, 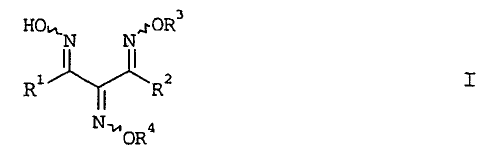 in der die Substituenten R¹ und R² gleich oder verschieden sein können und jeweils Cyano, C₁-C₄-Alkyl, C₁-C₄-Halogenalkyl und C₃-C₆-Cycloalkyl und R³ und R⁴ gleich oder verschieden sein können und jeweils C₁-C₄-Alkyl, C₁-C₄-Halogenalkyl, C₃-C₆-Cycloalkyl, C₁-C₄-Alkylcarbonyl und Aryl-C₁-C₄-alkyl bedeuten können,
durch folgende Reaktionsschritte in Wasser ohne Isolierung der Zwischenprodukte:
A) Nitrosierung von 1,3-Diketonen der Formel II 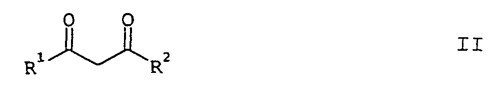 zu Monooximen der Formel III, 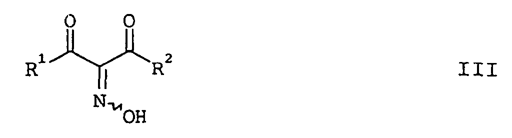
B) Oximierung von III mit Hydroxyaminderivaten der Formel IV
H₂N-OR^{3'} IV
in der R^{3'} für Wasserstoff oder eine Gruppe R³ steht, oder deren Säureadditionssalzen, zu Bisoximen der Formel V, 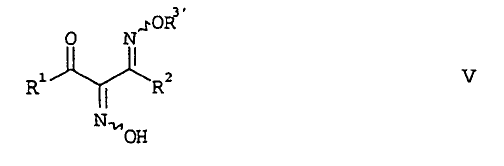
C) Alkylierung von V mit Alkylierungs- oder Acylierung von V mit Acylierungsmitteln zu Verbindungen der Formel VI und 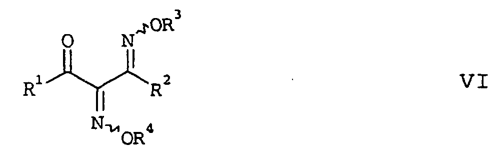
D) anschließender Oximierung von VI mit Hydroxylamin zu Verbindungen der Formel I.

2. Verfahren nach Anspruch 1, wobei die Nitrosierung in Schritt A unter sauren Bedingungen mit einem Alkali- oder Erdalkalinitrit, insbesondere Kalium- oder Natriumnitrit durchgeführt wird.

3. Verfahren nach einem der Ansprüche 1 oder 2, wobei in Formel I die Substituenten R³ und R⁴ gleich sind.

4. Verfahren nach Anspruch 3, wobei in Schritt B in Formel IV R^{3'} nicht Wasserstoff bedeutet.

5. Verfahren nach Anspruch 3, wobei in Schritt B Formel III mit Hydroxyamin zu Bisoximen der Formel V' 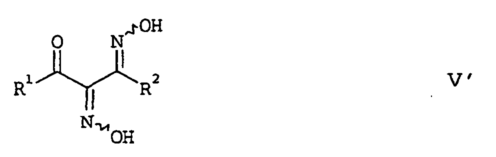 umgesetzt werden.

6. Verfahren nach einem der Ansprüche 1 oder 2, wobei in Formel I die Substituenten R³ und R⁴ verschieden sind.

7. Verfahren nach einem der Ansprüche 1 bis 3 oder 6, wobei in Formel IV R^{3'} für C₁-C₄-Alkyl, C₁-C₄-Halogenalkyl oder Aryl-C₁-C₄-alkyl steht.

8. Verfahren nach einem der Ansprüche 1 bis 7, wobei in Schritt. C Alkylierungsmittel, insbesondere Dialkylsulfate wie Dimethylsulfat verwendet werden.

9. Verfahren nach einem der Ansprüche 1 bis 7, wobei in Schritt C Acylierungsmittel, wie C₁-C₄-Carbonsäurehalogenide oder C₁-C₄-Carbonsäureanhydride verwendet werden.

10. Verfahren nach einem der Ansprüche 1 bis 8, wobei in Formel I die Substituenten R¹, R², R³ und R⁴ C₁-C₄-Alkyl bedeuten.

## Claims

1. A process for preparing trioxime derivatives of the formula I 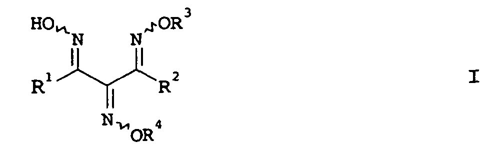 in which the substituents R¹ and R² can be identical or different and can in each case be cyano, C₁-C₄-alkyl, C₁-C₄-haloalkyl and C₃-C₆-cycloalkyl and R³ and R⁴ can be identical or different and can in each case be C₁-C₄-alkyl, C₁-C₄-haloalkyl, C₃-C₆-cycloalkyl, C₁-C₄-alkylcarbonyl and aryl-C₁-C₄-alkyl,
by the following reaction steps in water without isolation of the intermediates:
A) nitrosation of 1,3-diketones of the formula II 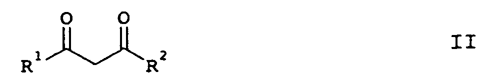 to give monooximes of the formula III, 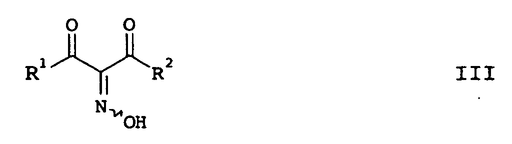
B) oximation of III with hydroxylamine derivatives of the formula IV
H₂N-OR^{3'} IV
in which R^{3'} is hydrogen or a group R³, or acid addition salts thereof, to give bisoximes of the formula V, 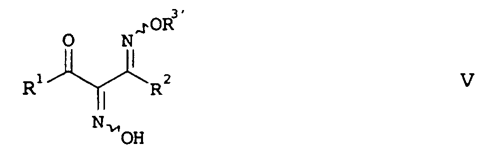
C) alkylation of V with alkylating agents or acylation of V with acylating agents to give compounds of the formula VI and 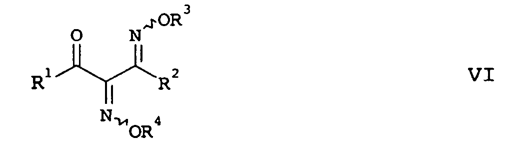
D) subsequent oximation of VI with hydroxylamine to give compounds of the formula I.

2. A process as claimed in claim 1 where the nitrosation in step A is carried out under acidic conditions using an alkali metal nitride or alkaline earth metal nitride, in particular potassium nitride or sodium nitride.

3. A process as claimed in claim 1 or 2 where the substituents R³ and R⁴ in formula I are identical.

4. A process as claimed in claim 3 where in step B R^{3'} in formula IV is not hydrogen.

5. A process as claimed in claim 3 where in step B the compound of the formula III is reacted with hydroxylamine to give bisoximes of the formula V'

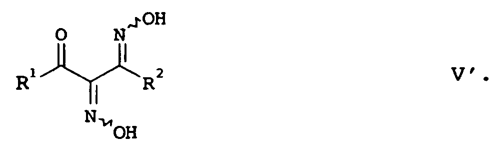

6. A process as claimed in claim 1 or 2 where the substituents R³ and R⁴ in formula I are different.

7. A process as claimed in any of claims 1 to 3 or 6 where R^{3'} in formula IV is C₁-C₄-alkyl, C₁-C₄-haloalkyl or aryl-C₁-C₄-alkyl.

8. A process as claimed in any of claims 1 to 7 where alkylating agents, in particular dialkyl sulfates such as dimethyl sulfate, are used in step C.

9. A process as claimed in any of claims 1 to 7 where acylating agents, such as C₁-C₄-carbonyl halides or C₁-C₄-carboxylic anhydrides, are used in step C.

10. A process as claimed in any of claims 1 to 8 where the substituents R¹, R², R³ and R⁴ in formula I are C₁-C₄-alkyl.

## Revendications

1. Procédé de préparation de dérivés de trioxime de la formule I : 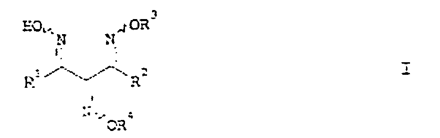 dans laquelle les substituants R¹ et R² peuvent être identiques ou différents et représenter chacun un groupe cyano, alkyle en C₁-C₄, halogénoalkyle en C₁-C₄ et cycloalkyle en C₃-C₆, et R³ et R⁴ peuvent être identiques ou différents et représenter chacun un groupe alkyle en C₁-C₄, halogénoalkyle en C₁-C₄, cycloalkyle en C₃-C₆, alkylcarbonyle en C₁-C₄ et aryle-alkyle en C₁-C₄,
par les étapes réactionnelles suivantes dans l'eau, sans isolement des produits intermédiaires :
A) une nitrosation de 1,3-dicétones de la formule II : 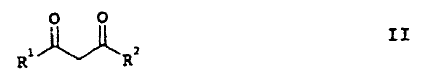 en monooximes de la formule III : 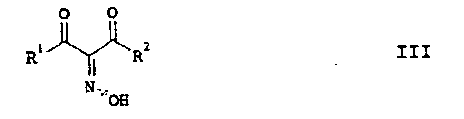
B) une oximation de III par des dérivés d'hydroxyamine de la formule IV :
H₂N-OR^{3'} IV
dans laquelle R^{3'} représente de l'hydrogène ou un groupe R³, ou par leurs sels d'addition d'acide, pour former des bisoximes de la formule V : 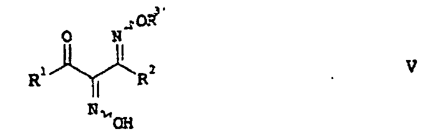
C) une alkylation de V avec des agents d'alkylation ou une acylation de V avec des agents d'acylation pour former des composés de la formule VI : 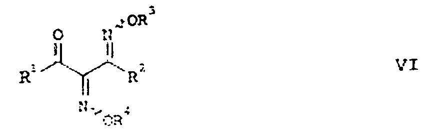 et
D) une oximation ultérieure de VI par de l'hydroxylamine pour former des composés de la formule I.

2. Procédé suivant la revendication 1, dans lequel la nitrosation de l'étape A est effectuée dans des conditions acides avec un nitrite alcalin ou alcalino-terreux, en particulier un nitrite de potassium ou de sodium.

3. Procédé suivant l'une des revendications 1 et 2, dans lequel, dans la formule I, les substituants R³ et R⁴ sont identiques.

4. Procédé suivant la revendication 3, dans lequel, dans l'étape B, R^{3,} ne représente pas de l'hydrogène dans la formule IV.

5. Procédé suivant la revendication 3, dans lequel, dans l'étape B, les composés de formule III sont, avec de l'hydroxyamine, convertis en bisoximes de la formule V' :

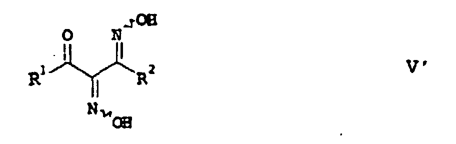

6. Procédé suivant l'une des revendications 1 et 2, dans lequel, dans la formule I, les substituants R³ et R⁴ sont différents.

7. Procédé suivant l'une des revendications 1 à 3 ou 6, dans lequel, dans la formule IV, R^{3'} représente un groupe alkyle en C₁-C₄, halogénoalkyle en C₁-C₄ ou aryle-alkyle en C₁-C₄.

8. Procédé suivant l'une des revendications 1 à 7, dans lequel, dans l'étape C, on utilise des agents d'alkylation, en particulier des sulfates de dialkyle, comme du sulfate de diméthyle.

9. Procédé suivant l'une des revendications 1 à 7, dans lequel, dans l'étape C, on utilise des agents d'acylation, tels que des halogénures d'acide carboxylique en C₁-C₄ ou des anhydrides d'acide carboxylique en C₁-C₄.

10. Procédé suivant l'une des revendications 1 à 8, dans lequel, dans la formule I, les substituants R¹, R², R³ et R⁴ représentent un groupe alkyle en C₁-C₄.
